# DEMANDE DE BREVET EUROPEEN

(11) **EP 1 559 397 A1**
(43) Date de publication de la demande: **03.08.2005**
(21) Numéro de dépôt: 05290123.8
(22) Date de dépôt: 20.01.2005
(51) Int. Cl.: A61K 7/06, A61K 7/48

(54) **Procédé de préparation d'une composition de traitement cosmétique à partir de fluide sous pression, de céramides, de cires et/ou de silicones**

(30) Priorité: 29.01.2004 FR 0400845
(71) Demandeur: L'OREAL, 75008 Paris (FR)
(72) Inventeur: De la Mettrie, Roland, 78110 Le Vesinet (FR)
(74) Mandataire: Casalonga, Axel

(57) **Abrégé**

La présente invention concerne un procédé de préparation d'une composition de traitement cosmétique des matières kératiniques, par percolation de fluide comprenant au moins de la vapeur d'eau, sous une pression allant de 3 à 30 bars, au travers d'au moins un agent de conditionnement siliconé ou non, sous forme solide ou pâteuse, choisi parmi les alcools gras linéaires ou ramifiés en C₁₄₋₅₀, les hydrocarbures linéaires ou ramifiés, d'origine minérale ou synthétique, les cires, les composés de type céramide, et les gommes ou résines de silicone organomodifiées ou non.

L'invention concerne aussi un dispositif de conditionnement d'une composition contenant un tel agent ainsi que le procédé de traitement cosmétique mettant en oeuvre une telle composition.

## Description

La présente invention a pour objet un procédé de préparation d'une composition cosmétique destinée au traitement des matières kératiniques, en particulier de la peau et des fibres kératiniques humaines telles que les cheveux, ainsi qu'un procédé de traitement cosmétique des matières kératiniques à partir de cette composition.

En cosmétique on cherche toujours à améliorer les propriétés cosmétiques des matières kératiniques, par exemple des cheveux sensibilisés, i.e. qui sont abîmés ou fragilisés sous l'action chimique des agents atmosphériques et/ou des traitements capillaires tels que permanentes, teintures ou décolorations. On peut alors leur appliquer des compositions de traitement cosmétique comprenant des agents de conditionnement siliconés ou non tels que des cires, des céramides ou des silicones, destinés à réparer ou limiter les effets néfastes ou indésirables produits par les différents traitements ou agressions que subissent, de manière plus ou moins répétée, les matières kératiniques. Ces agents de conditionnement peuvent également améliorer les propriétés cosmétiques des cheveux naturels.

Les agents de conditionnement tels que des cires, des céramides ou des silicones, confèrent aux cheveux secs ou mouillés, par exemple, un effet coiffant et fixant, une facilité de démêlage, une douceur et/ou un lissage.

Ces agents de conditionnement confèrent également à la peau des propriétés cosmétiques telles qu'une bonne hydratation et une bonne nutrition. En outre, les cires permettent de lisser et d'estomper les rides.

Cependant, les compositions de traitement cosmétique contenant de tels agents de conditionnement sont généralement des compositions aqueuses dans lesquelles lesdits agents doivent être solubilisés. Le manque de solubilité de ces composés diminue le pouvoir conditionnant de ces compositions. En outre, ce critère de solubilité réduit le nombre de cires, de céramides et de silicones que l'on peut utiliser pour le traitement cosmétique des matières kératiniques. Ceci est particulièrement le cas des composés à point de fusion élevé.

La Demanderesse a découvert de manière surprenante qu'en utilisant un nouveau procédé de préparation d'une composition de traitement cosmétique des matières kératiniques, on pouvait obtenir en un temps très court, inférieur à 2 minutes, des compositions plus ou moins concentrées en agent(s) de conditionnement selon le besoin, notamment sans conservateur, permettant de surmonter les problèmes de solubilité exposés ci-dessus.

Ce procédé est mis en oeuvre simplement et est approprié au besoin du consommateur. On fait passer un fluide, dont la température est de préférence supérieure ou égale à 30 °C, sous pression, pendant un laps de temps très court, inférieur à 1 minute, à travers au moins un agent de conditionnement sous forme solide ou pâteuse, de préférence solide, et encore plus préférentiellement pulvérulente.

Il permet aussi d'utiliser sous forme anhydre des agents de conditionnement instables dans des compositions aqueuses soit parce qu'ils réagissent avec l'eau, soit parce qu'ils réagissent en solution aqueuse avec des composés qui ne réagissent pas avec eux dans une composition anhydre.

Les compositions préparées selon ce procédé peuvent présenter une stabilité au stockage limitée, ce qui n'est pas ici un inconvénient puisque le procédé conduit à une composition prête à l'emploi, destinée à être utilisée rapidement après sa préparation, par exemple dans les 5 minutes suivant sa préparation, notamment après refroidissement jusqu'à une température acceptable pour les matières kératiniques, de préférence inférieure à 60 °C, mieux inférieure à 50 °C. La composition peut être également utilisée jusqu'à une semaine après sa préparation, selon la vitesse de dégradation de l'agent de conditionnement utilisé.

Etant donné le temps de préparation très court, les compositions de traitement cosmétique peuvent être préparées "à la demande" en mélangeant différents composés actifs en cosmétique selon les propriétés cosmétiques recherchées.

Selon un autre mode de réalisation, les agents conditionnement pouvant être conditionnés dans un dispositif prêt-à-l'emploi, il n'est pas nécessaire de déterminer au préalable les concentrations desdits agents en solution, ce qui limite les erreurs de mesure de l'utilisateur.

En outre, le procédé selon l'invention permet d'éviter l'utilisation de flacons multi-compartiments, ce qui rend le procédé particulièrement économique et plus sûr pour l'utilisateur.

La composition ainsi obtenue peut être utilisée seule ou en mélange avec une autre composition.

Un avantage supplémentaire de ce procédé de préparation est l'obtention de compositions présentant de meilleures propriétés cosmétiques. En particulier, les fibres kératiniques traitées avec une composition obtenue par le procédé selon l'invention présentent des propriétés de conditionnement améliorées, et notamment de démêlage, de lissage, de brillance et de douceur au toucher, et la peau ainsi traitée présente une hydratation et un lissage des rides améliorés.

L'invention a donc pour objet un procédé de préparation d'une composition de traitement cosmétique des matières kératiniques comprenant une étape de percolation d'un fluide sous une pression d'au moins 3 bars au travers d'au moins un agent de conditionnement siliconé ou non, sous forme solide ou pâteuse.

Un autre objet de l'invention est une composition susceptible d'être obtenue par le procédé selon l'invention.

L'invention a également pour objet l'utilisation de la composition obtenue selon le procédé de l'invention, pour le traitement cosmétique des matières kératiniques, et notamment le conditionnement des cheveux.

L'invention a encore pour objet un dispositif de conditionnement permettant de mettre en oeuvre le procédé de préparation de la présente invention.

D'autres objets, caractéristiques, aspects et avantages de l'invention apparaîtront encore plus clairement à la lecture de la description et de l'exemple qui suit.

Selon l'invention, le procédé de préparation d'une composition de traitement cosmétique des matières kératiniques comprend une étape de percolation de fluide, de préférence à une température supérieure ou égale à 30 °C, mieux encore allant de 30 à 150 °C, et encore plus préférentiellement de 40 à 120 °C, sous une pression d'au moins 3 bars (3.10⁵ Pa) au travers d'au moins un agent de conditionnement siliconé ou non tel que décrit ci-dessous, qui est de préférence insoluble dans l'eau et non cationique, sous forme solide ou pâteuse.

La percolation est un mouvement de fluide à travers un milieu poreux permettant le passage du fluide, sous l'action ou l'effet de la pression.

Le fluide comprend au moins de la vapeur d'eau. Il peut être constitué par de la vapeur d'eau éventuellement accompagnée d'eau liquide, ou par un mélange de vapeur d'eau éventuellement accompagnée d'eau liquide, et d'un ou plusieurs solvants liquides et/ou gazeux cosmétiquement acceptables. De préférence, le fluide est de la vapeur d'eau pouvant être accompagnée d'eau liquide.

A titre de solvant organique, on peut citer, par exemple, les alcools inférieurs en C₁-C₄ tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Par "insoluble dans l'eau", on entend tout composé qui a une concentration supérieure ou égale à 0,1% en poids dans l'eau à 25°C, ne forment pas à l'oeil nu une solution isotrope transparente.

L'agent de conditionnement est sous forme solide ou pâteuse, de préférence sous forme solide, et encore plus préférentiellement pulvérulente.

Par «forme pâteuse» au sens de la présente invention, on entend une consistance intermédiaire entre une phase solide et une phase liquide. La viscosité de cette phase pâteuse est de préférence supérieure à 0,1 Pa.s, et encore plus préférentiellement supérieure à 1 Pa.s, ceci à 25°C avec un taux de cisaillement de 10 s⁻¹.

Par "matières kératiniques", on entend la peau, les lèvres, et/ou les phanères tels que les ongles et les fibres kératiniques, par exemple les cils, les sourcils et les cheveux.

Le procédé de la présente invention peut être mis en oeuvre à partir d'un dispositif classique permettant de générer un fluide sous pression, à une température de préférence supérieure ou égale à 30 °C, mieux encore allant de 30 à 150 °C, et encore plus préférentiellement de 40 à 120 °C. Un tel dispositif comprend une chambre résistant à la pression, équipée d'un bloc thermique, ainsi qu'un circuit d'acheminement du fluide vers l'agent de conditionnement utilisé dans l'invention.

Selon un autre mode de réalisation, le dispositif comprend de plus un réservoir de liquide(s) ainsi qu'une pompe permettant l'acheminement du ou des liquides vers la chambre.

Le liquide contenu dans le réservoir est soit de l'eau, soit un mélange d'eau et d'un ou plusieurs solvants cosmétiquement acceptables. De préférence, le liquide est de l'eau.

Un dispositif particulièrement utile pour la mise en oeuvre du procédé de la présente invention est une machine à café du type "expresso". De telles machines sont bien connues de la technique. Par exemple, ces machines sont décrites dans les brevets AT 168405, US 2688911, DE 32433870 et IT 1265636.

Selon un mode de réalisation particulier de l'invention, l'étape de percolation est mise en oeuvre avec un fluide à une température supérieure ou égale à 30 °C, mieux encore allant de 30 à 150 °C, et encore plus préférentiellement de 40 à 120 °C, sous une pression allant de 3 à 30 bars (3.10⁵ à 3.10⁶ Pa), de préférence d'au moins 4 bars (4.10⁵ Pa), mieux encore supérieure ou égale à 10 bars (10⁶ Pa), et tout particulièrement allant de 10 à 30 bars (10⁶ à 3.10⁶ Pa).

Une composition cosmétique comprenant au moins un agent de conditionnement tel que décrit ci-dessous, de préférence sous forme solide ou pâteuse, peut être utilisée directement dans le dispositif générant le fluide sous pression dans un récipient destiné à cet usage. Elle peut aussi être conditionnée dans un dispositif de conditionnement particulier comprenant un logement fermé délimité par au moins une paroi au moins en partie perméable au fluide sous une pression d'au moins 3 bars, de préférence allant de 3 à 30 bars, mieux encore d'au moins 4 bars, plus particulièrement supérieure ou égale à 10 bars, et tout particulièrement allant de 10 à 30 bars. De tels dispositifs sont, par exemple, décrits dans les demandes de brevets WO 00/56629, EP512470, US 5897899 ou WO 99/03573. Ces dispositifs de conditionnement sont en général étanches à l'air, l'humidité et/ou la lumière.

Selon un mode de réalisation particulier, le logement est délimité par deux feuilles scellées. Selon un autre mode de réalisation, le logement est délimité par une barquette fermée par un couvercle.

Ces dispositifs peuvent être fabriqués à partir de matériaux tissés ou non tissés, en matière plastique ou végétale, par exemple en cellulose, en métal tel que l'aluminium ou en composite. De tels dispositifs sont par exemple décrits dans les demandes de brevets WO 00/56629, EP512470, US 5897899 ou WO 99/03573.

Les agents de conditionnement siliconés ou non, utilisés dans l'invention sont de préférence insolubles dans l'eau et/ou non cationiques. Ils sont notamment choisis parmi :
- les alcools gras linéaires ou ramifiés ayant de 14 à 50 atomes de carbone tels que les alcools cétylique, myristique, stéarylique, palmitique, béhénique et eicosanoique ;
- des hydrocarbures linéaires ou ramifiés, d'origine minérale
ou synthétique tels que les cires de paraffine et leurs dérivés, les poly(α-oléfines), la vaseline, les polydécènes, et les polyisobutènes hydrogénés ;
- les cires,
- les composés de type céramide, et
- les gommes ou résines de silicone organomodifiées ou non.

Des agents de conditionnement particulièrement adaptés à l'invention sont les cires.

Une cire, au sens de la présente invention, est un composé gras lipophile, solide ou pâteux à température ambiante (20 à 25°C), à changement d'état solide/liquide réversible, ayant une température de fusion supérieure à 25°C et pouvant aller jusqu'à 200°C, généralement avec une dureté supérieure à 0,5 MPa pour les solides et une dureté comprise entre 0,001 et 0,5 MPa pour les pâtes, et présentant à la température de fusion une organisation cristalline anisotrope.

A titre de cires insolubles dans l'eau, utilisables dans la présente invention, on peut notamment citer les cires d'origine animale, végétale, minérale ou synthétique telles que la cire d'abeille, le spermaceti, les cires fluorées et perfluorées, les cires de lanoline, de lanoline hydrogénée et de lanoline acétylée ; les cires de Candellila, d'Ouricury, de Carnauba, du Japon, le beurre de cacao, les cires de fibres de liège ou de canne à sucre, la cire de son de riz, la cire de sapin, la cire de coton ; les cires microcristallines, la cire de paraffine, le pétrolatum, la vaseline, l'ozokérite, la cire de montan ; les huiles hydrogénées ayant une température de fusion supérieure à environ 40°C, comme l'huile de jojoba hydrogénée ; les cires de polyéthylène et les cires obtenues par synthèse de Fischer-Tropsch.

Par composés de type céramide au sens de la présente invention, on entend les céramides et/ou les glycocéramides et/ou les pseudocéramides et/ou les néocéramides, naturels ou synthétiques.

Des composés de type céramide sont par exemple décrits dans les demandes de brevet DE4424530, DE4424533, DE4402929, DE4420736, WO95/23807, EP-A-0646572, WO95/16665, FR-2 673 179, EP-A-0227994 et WO 94/07844, WO94/24097, WO94/10131.

Les composés de type céramide utilisables dans la présente invention répondent par exemple à la formule générale (I) suivante : 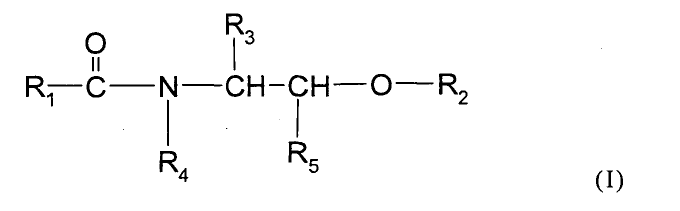 dans laquelle :
- R₁ désigne :
   - soit un radical hydrocarboné, linéaire ou ramifié, saturé ou insaturé, en C₁-C₅₀, de préférence en C₅-C₅₀, ce radical pouvant être substitué par un ou plusieurs groupements hydroxyle éventuellement estérifié par un acide R₇COOH, R₇ étant un radical hydrocarboné, saturé ou insaturé, linéaire ou ramifié, éventuellement mono ou polyhydroxylé, en C₁-C₃₅ , le ou les substituants hydroxyle du radical R₇ pouvant être estérifiés par un acide gras saturé ou insaturé, linéaire ou ramifié, éventuellement mono ou polyhydroxylé, en C₁-C₃₅;
   - soit un radical R₁"-(NR°-CO)-R₁'-, R° désignant un atome d'hydrogène ou un radical hydrocarboné en C₁-C₂₀ mono ou polyhydroxylé, préférentiellement monohydroxylé, R₁' et R₁" étant des radicaux hydrocarbonés dont la somme des atomes de carbone va de 9 à 30, R₁' étant un radical divalent.
   - soit un radical R₈-O-CO-(CH₂)ₚ-, R₈ désignant un radical hydrocarboné en C₁-C₂₀, p étant un entier variant de 1 à 12.
- R₂ est choisi parmi un atome d'hydrogène, un radical de type saccharidique, en particulier un radical (glycosyle)ₙ, (galactosyle)ₘ ou sulfogalactosyle, un résidu de sulfate ou de phosphate, un radical phosphoryléthylamine et un radical phosphoryléthylammonium, dans lesquels n est un entier variant de 1 à 4 et m est un entier variant de 1 à 8 ;
- R₃ désigne un atome d'hydrogène ou un radical hydrocarboné en C₁-C₃₃, saturé ou insaturé, mono ou polyhydroxylé ou non hydroxylé, le ou les substituants hydroxyle pouvant être estérifiés par un acide minéral ou un acide R₇COOH, R₇ ayant les mêmes significations que ci-dessus, le ou les substituants hydroxyle pouvant être éthérifiés par un radical (glycosyle)ₙ, (galactosyle)ₘ, sulfogalactosyle, phosphoryléthylamine ou phosphoryléthylammonium, R₃ pouvant également être substitué par un ou plusieurs radicaux alkyle en C₁-C₁₄ ; de préférence, R₃ désigne un radical α-hydroxyalkyle en C₁₅-C₂₆, le groupement hydroxyle étant éventuellement estérifié par un α-hydroxyacide en C₁₆-C₃₀ ;
- R₄ désigne un atome d'hydrogène, un radical méthyle, éthyle, un radical hydrocarboné en C₃-C₅₀, saturé ou insaturé, linéaire ou ramifié, éventuellement hydroxylé ou un radical -CH₂-CHOH-CH₂-O-R₆ dans lequel R₆ désigne un radical hydrocarboné en C₁₀-C₂₆ ou un radical R₈-O-CO-(CH₂)ₚ-, R₈ désignant un radical hydrocarboné en C₁-C₂₀, p étant un entier variant de 1 à 12,
- R₅ désigne un atome d'hydrogène ou un radical hydrocarboné en C₁-C₃₀ saturé ou insaturé, linéaire ou ramifié, éventuellement mono ou polyhydroxylé, le ou les substituants hydroxyle pouvant être éthérifiés par un radical (glycosyle)ₙ, (galactosyle)ₘ, sulfogalactosyle, phosphoryléthylamine ou phosphoryléthylammonium,
   sous réserve que lorsque R₃ et R₅ désignent un atome d'hydrogène ou lorsque R₃ désigne un atome d'hydrogène et R₅ désigne un radical méthyle alors R₄ ne désigne pas un atome d'hydrogène, un radical méthyle ou éthyle.

Parmi les composés de formule (I), on préfère les céramides et/ou glycocéramides dont la structure est décrite par DOWNING dans Journal of Lipid Research Vol. 35, 2060-2068, 1994, ou ceux décrits dans la demande de brevet français FR-2 673 179.

Les composés de type céramide plus particulièrement préférés selon l'invention sont les composés de formule (I) pour lesquels R₁ désigne un radical alkyle saturé ou insaturé dérivé d'acides gras en C₁₄-C₂₂ éventuellement hydroxylé; R₂ désigne un atome d'hydrogène ; et R₃ désigne un radical linéaire en C₁₁₋₁₇ éventuellement hydroxylé et de préférence en C₁₃₋₁₅.

De tels composés sont par exemple :
- le 2-N-linoléoylamino-octadécane-1,3-diol,
- le 2-N-oléoylamino-octadécane-1,3-diol,
- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1,3-diol,
- le 2-N-stéaroyl amino-octadécane-1,3,4 triol et en particulier la N-stéaroyl phytosphingosine,
- le 2-N-palmitoylamino-hexadécane-1,3-diol
ou les mélanges de ces composés.

On peut également utiliser les composés de formule (I) pour lesquels R₁ désigne un radical alkyle saturé ou insaturé dérivé d'acides gras en C₁₂-C₂₂ ; R₂ désigne un radical galactosyle ou sulfogalactosyle ; et R₃ désigne un radical hydrocarboné en C₁₂-C₂₂, saturé ou insaturé et de préférence un groupement - CH(OH)-CH=CH-(CH₂)₁₂-CH₃.

A titre d'exemple, on peut citer le produit constitué d'un mélange de glycocéramides, vendu sous la dénomination commerciale GLYCOCER par la société WAITAKI INTERNATIONAL BIOSCIENCES.

On peut également utiliser les composés de formule (I) décrits dans les demandes de brevet EP-A-0227994 et WO 94/07844.

De tels composés sont par exemple le QUESTAMIDE H (bis-(N-hydroxyéthyl N-cétyl) malonamide) vendu par la société QUEST.

On peut également utiliser le N-docosanoyl N-méthyl-D-glucamine décrit dans la demande de brevet WO94/24097.

D'autres agents de conditionnement selon l'invention sont les gommes ou résines de silicone organomodifiées ou non.

Les silicones (ou polyorganosiloxanes) non organomodifiées utilisables dans la présente invention, sont des polysiloxanes comportant sur les atomes de silicium des groupements hydroxy ou des groupements alkyles, alcényles (en particulier vinyle) ou aryles, ces groupements étant substitués ou non substitués. Elles présentent de préférence une viscosité allant de 50 000 à 750 000 mm²/s à 25°C.

Les organopolysiloxanes sont définis plus en détail dans l'ouvrage de Walter NOLL "Chemistry and Technology of Silicones" (1968) Academic Press.

A titre d'exemples de résines ou de gommes de silicone non organomodifiées, on peut citer :
(i) les polydialkylsiloxanes ;
(ii) les polydiarylsiloxanes ;
(iii) les polyalkylarylsiloxanes ;
(iv) ou leurs mélanges.

Les radicaux alkyles comportent notamment de 1 à 10 atomes de carbone et désignent en particulier méthyle. Les radicaux aryles désignent plus particulièrement phényle.

Ces silicones sont plus particulièrement choisies parmi les polydialkylsiloxanes parmi lesquels on peut citer principalement les polydiméthylsiloxanes à groupements terminaux triméthylsilyle ou diméthylsilanol.

Parmi les polydialkylsiloxanes, on peut citer principalement :
- les polydiméthylsiloxanes linéaires à groupements terminaux triméthylsilyle et ayant une viscosité de 50 000 à 750 000 mm²/s à 25°C, comme par exemple, et à titre non limitatif, les silicones SILBIONE® de la série 70047 commercialisées par RHODIA CHIMIE, notamment l'huile SILBIONE® 70047 V 500 000 de RHODIA CHIMIE ou le polydiméthylsiloxane de viscosité 300 000 mm²/s (300 000 cSt), vendu sous la dénomination DC 200 fluid 300 000 par la société DOW CORNING , les silicones de la série 200 de la société DOW CORNING telles que plus particulièrement la DC200 de viscosité 60 000 mm²/s (60 000 cSt) ;
- les polydiméthylsiloxanes linéaires à groupements terminaux hydroxydiméthylsilyle (Dimethiconol selon la dénomination CTFA).

Dans cette classe de polyalkylsiloxanes, on peut également mentionner les polyalkylsiloxanes vendus par la société GOLDSCHMIDT sous les dénominations commerciales ABILWAX® 9800 et ABILWAX® 9801 qui sont des polyalkyl(C₁-C₂₀)siloxanes.

Les polyalkylarylsiloxanes sont particulièrement choisis parmi les polydiméthyl-méthylphénylsiloxanes, les polydiméthyl-diphényl-siloxanes linéaires et/ou ramifiés de viscosité de 50 000 à 1 000 000 mm²/s à 25°C.

Les gommes de silicone, conformes à l'invention, sont plus particulièrement des polyorganosiloxanes de masse moléculaire moyenne en nombre comprise entre 200 000 et 1 000 000. On cite, par exemple, les composés suivants :
- polydiméthylsiloxane,
- poly[(diméthylsiloxane)/(méthylvinylsiloxane)],
- poly[(diméthylsiloxane)/(diphénylsiloxane)],
- poly[(diméthylsiloxane)/(phénylméthylsiloxane)],
- poly[(diméthylsiloxane)/(diphénylsiloxane)/(méthylvinylsilo-xane)].

Un produit plus particulièrement utilisable conformément à l'invention est la gomme SE 30 de GENERAL ELECTRIC correspondant à une diméthicone, ayant un poids moléculaire moyen en nombre de 500 000.

Les résines d'organopolysiloxanes utilisables conformément à l'invention sont les composés siloxaniques réticulés renfermant les motifs :

R₂SiO_{2/2}, R₃SiO_{1/2}, RSiO_{3/2} et SiO_{4/2}

dans lesquels R représente un groupement hydrocarboné comportant de 1 à 16 atomes de carbone, ou un groupement phényle. Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthyl-siloxysilicate commercialisées notamment sous les dénominations X22-4914, X21-5034 et X21-5037 par la société SHIN-ETSU.

Les silicones organomodifiées utilisables conformément à l'invention sont des silicones telles que définies précédemment et comportant dans leur structure un ou plusieurs groupements organofonctionnels, de préférence non cationiques, fixés par l'intermédiaire d'un radical hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄,
- des groupements thiols ;
- des groupements alcoxylés tels que les produits ABIL WAX 2428, 2434 et 2440 vendus par la société GOLDSCHMIDT ;
- des groupements hydroxylés, comme les polyorganosiloxanes à fonction hydroxyalkyle décrits dans la demande de brevet français FR-A-85 16334 ;
- des groupements acyloxyalkyle en C₃-C₃₀ ;
- des groupements anioniques du type carboxylique tels qu'un groupement béhénate ;
- des groupements polyesters, tels que les polydiméthylsiloxanes à groupements α,ω-hydroxypolycaprolactone, les polydiméthylsiloxanes-α,ω-silanols à groupements jasminate ; et/ou
- des groupements hydroxyacylamino.

On peut également utiliser à titre de silicones organomodifiées dans le procédé de l'invention, des copolymères blocs ayant un bloc linéaire polysiloxane-polyoxyalkylène de type (A-B)ₙ, et répondant de préférence à la formule générale suivante :

([ Y (R₂SiO)ₐ R'₂SiYO] [(CₙH₂ₙO)_{b}])_{c} (II)

dans laquelle :
- R et R', identiques ou différents, représentent un radical hydrocarboné monovalent,
- n est un nombre entier allant de 2 à 4,
- a est un nombre entier supérieur ou égal à 5, de préférence compris entre 5 et 200, et encore plus particulièrement entre 5 et 100.
- b est un nombre entier supérieur ou égal à 4, de préférence compris entre 4 et 200, et encore plus particulièrement entre 5 et 100.
- c est un nombre entier supérieur ou égal à 4, de préférence compris entre 4 et 1000, et encore plus particulièrement entre 5 et 300.
- Y représente un groupe organique divalent qui est lié à l'atome de silicium adjacent par une liaison carbone-silicium et à un bloc polyoxyalkylène par un atome d'oxygène,
- le poids moléculaire moyen de chaque bloc siloxane est compris entre environ 400 et environ 10 000, celui de chaque bloc polyoxyalkylène étant compris entre environ 300 et environ 10 000,
- les blocs siloxane représentent de 10 % environ à 95 % environ en poids du copolymère bloc,
- le poids moléculaire moyen en poids du copolymère bloc étant d'au moins 3 000, et de préférence compris entre 5000 et 1000000 et encore plus particulièrement entre 10000 et 200000.

R et R' sont préférentiellement choisis parmi le groupe comprenant les radicaux alkyles comme, par exemple, les radicaux méthyle, éthyle, propyle, butyle, pentyle, hexyle, octyle, décyle, dodécyle, les radicaux aryles comme, par exemple, phényle et naphtyle, les radicaux aralkyles comme, par exemple, benzyle, phényléthyle, les radicaux tolyle, xylyle et cyclohexyle.

Y est de préférence -R"-, -R"-CO-, -R"-NHCO-, -R"-NH-CO-NH-R"'-NHCO―, -R"-OCONH-R"'-NHCO-, où R" est un groupe alkylène divalent comme, par exemple, un groupe éthylène, propylène
ou butylène et R"' est un groupe alkylène divalent ou un groupe arylène divalent comme -C₆H₄-, -C₆H₄-C₆H₄-, -C₆H₄-CH₂-C₆H₄-, -C₆H₄-C(CH₃)₂-C₆H₄-.

Encore plus préférentiellement, Y représente un radical alkylène divalent, plus particulièrement le radical -CH₂-CH₂-CH₂- ou le radical -C₄H₈-.

La préparation des copolymères blocs mis en oeuvre dans le cadre de la présente invention est décrite dans la demande européenne EP 0 492 657 A1.

Selon l'invention, on peut encore utiliser comme silicone organomodifiée, des polyuréthanes siliconés tels que ceux décrits dans les demandes de brevet EP 0 751 162, EP 0 619 111 et EP1025833.

Selon l'invention, on peut également utiliser des silicones organomodifiées comprenant une portion polysiloxane et une portion constituée d'une chaîne organique non-siliconée, l'une des deux portions constituant la chaîne principale du polymère, l'autre étant greffée sur la dite chaîne principale. Ces polymères sont par exemple décrits dans les demandes de brevet EP-A-412 704, EP-A-412 707, EP-A-640 105, WO 95/00578, EP-A-582 152 et WO 93/23009 et les brevets US 4,693,935, US 4,728,571 et US 4,972,037. Ces polymères sont de préférence anioniques ou non ioniques.

De tels polymères sont, par exemple, les copolymères susceptibles d'être obtenus par polymérisation radicalaire à partir du mélange de monomères constitué par :
a) 50 à 90% en poids d'acrylate de tertiobutyle ;
b) 0 à 40% en poids d'acide acrylique ;
c) 5 à 40% en poids de macromère siliconé de formule : 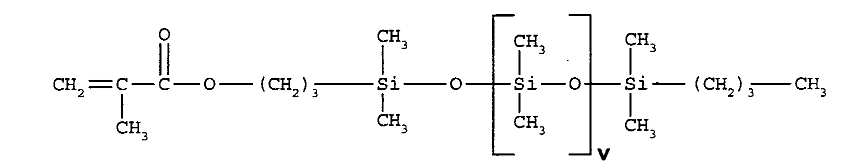 avec v étant un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Les polyorganosiloxanes particulièrement préférés conformément à l'invention sont :
- les silicones choisies dans la famille des polydiméthylsiloxanes à groupements terminaux triméthylsilyle telles que les huiles de la séries DC200 de DOW CORNING de viscosité 60 000 mm²/s (60 000 cSt), des séries SILBIONE® 70047 et 47 et plus particulièrement l'huile SILBIONE® 70 047 V 500 000 commercialisées par la société RHODIA CHIMIE, ou l'huile de silicone AK 300.000 de la société WACKER, les polydiméthylsiloxanes à groupements terminaux diméthylsilanol tels que les diméthiconols, ou les polyalkylarylsiloxanes tels que l'huile SILBIONE 70641 V 200 commercialisée par la société RHONE POULENC ; et
- la résine d'organopolysiloxane commercialisée sous la dénomination DOW CORNING 593.

Les agents de conditionnement de l'invention peuvent être mis en oeuvre en mélange avec un ou plusieurs adjuvants solides ou pâteux, et de préférence pulvérulents. Les adjuvants peuvent être choisis parmi les argiles, les sels, les tensioactifs anioniques, non ioniques, cationiques ou zwittérioniques, les épaississants naturels ou de synthèse, l'amidon éventuellement modifié, les billes de verre, la silice, le nylon, l'alumine, le dioxyde de titane, les zéolithes, le poly(méthacrylate de méthyle) (PMMA), le chitosane, la maltodextrine, la cyclodextrine, les mono- ou disaccharides comme le glucose, le saccharose, le sorbitol et le fructose, l'oxyde de zinc, de zirconium, les silicabades, le talc, l'acide polyaspartique, les borosilicates notamment de calcium, le polyéthylène, le coton, le polytétrafluoroéthylène (PTFE), la cellulose et ses dérivés, les composés superabsorbants, les carbonates de magnésium ou de calcium, les grains de maïs, le polyacrylamide, l'hydroxyapatite poreux, la soie, le collagène, la sciure de bois, la poudre de fucus, les farines ou extraits de blé, de riz, de pois, de lupin, de soja ou d'orge, la polyvinylpyrrolidone réticulée, l'alginate de calcium, le charbon actif, les particules de poly(chlorure de vinylidène/acrylonitrile), notamment celles commercialisées sous la dénomination générale d'« Expancel® » par la société AKZO NOBEL sous les références particulières « Expancel® WE» ou « DE » Expancels, et leurs mélanges.

Lorsqu'un ou plusieurs adjuvants sont présents, le ou lesdits agents de conditionnement de l'invention sont présents de préférence en une quantité allant de 0,5 à 99% en poids, mieux encore de 1 à 80 % en poids, et encore plus préférentiellement de 2 à 60 % en poids par rapport au poids total agent(s) de conditionnement et adjuvant(s) sous forme solide ou pâteuse.

Lorsque des plantes ou extraits de plantes sont utilisés dans le procédé selon l'invention, ils peuvent être soumis, avant la percolation, à un prétraitement tel qu'une torréfaction, un cryobroyage, ou une lyophilisation.

La composition de traitement cosmétique des matières kératiniques obtenue selon le procédé de l'invention contient outre le
ou les agents de conditionnement et le(s) composant(s) du fluide, à savoir l'eau et/ou le(s) solvant(s) cosmétiquement acceptable(s), éventuellement tout ou partie du ou des adjuvants présents dans le mélange solide ou pâteux.

L'invention concerne également une composition susceptible d'être obtenue par le procédé selon l'invention, la composition particulièrement préférée étant exempte d'agents conservateurs.

A partir du procédé de préparation de l'invention, on obtient une composition de traitement cosmétique des matières kératiniques qui peut être appliquée directement sur les matières kératiniques, ou qui peut être mélangée à un milieu cosmétiquement acceptable, ou encore au moins un additif classiquement utilisé en cosmétique pourra y être ajouté par un opérateur. On peut également mélanger au moins deux compositions obtenues par le procédé de l'invention. La composition de traitement cosmétique des matières kératiniques résultant éventuellement de mélange(s) et/ou addition(s) indiqués ci-dessus, sera dénommée ci-après composition finale de traitement cosmétique ou composition finale.

Un mode de réalisation particulier de l'invention consiste à appliquer la composition obtenue par l'intermédiaire d'un dispositif ne nécessitant pas d'intervention humaine et équipé éventuellement d'un moyen de refroidissement.

La quantité dudit agent ou desdits agents de conditionnement, présents dans la composition finale de traitement cosmétique est en général comprise entre 0,001 à 50% en poids environ, de préférence entre 0,005 et 30% en poids, et encore plus préférentiellement entre 0,01 et 20% en poids par rapport au poids total de la composition finale de traitement cosmétique.

Lorsque la composition de traitement cosmétique obtenue par le procédé de la présente invention est mélangée à un milieu cosmétiquement acceptable, un tel milieu est généralement constitué par de l'eau ou par un mélange d'eau et d'au moins un solvant organique pour solubiliser les composés qui ne seraient pas suffisamment solubles dans l'eau.

Par "cosmétiquement acceptable", on entend un milieu compatible avec les matières kératiniques et notamment la peau, les lèvres et/ou les phanères, et qui en outre, présente un aspect, un toucher, une odeur et éventuellement un goût agréable pour l'utilisateur.

A titre de solvant organique, on peut citer, par exemple, les alcools inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ, et encore plus préférentiellement entre 5 et 30 % en poids environ par rapport au poids total de la composition finale de traitement cosmétique.

Au moins un additif classiquement utilisé en cosmétique peut être également ajouté dans les compositions de traitement cosmétique obtenues selon le procédé de la présente invention. A titre d'exemples de tels additifs, on peut citer des agents tensioactifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement différents de ceux décrits ci-dessus tels que, par exemple, des huiles de silicone, des agents filmogènes, des agents conservateurs, des agents opacifiants, des pigments nacrés ou colorés.

Les additifs ci-dessus sont en général présents en une quantité comprise pour chacun d'eux entre 0,01 et 20 % en poids par rapport au poids de la composition finale.

Bien entendu, l'homme de l'art veillera à choisir ce ou ces éventuels composés complémentaires de manière telle que les propriétés avantageuses attachées intrinsèquement à la composition de traitement cosmétique conforme à l'invention ne soient pas, ou substantiellement pas, altérées par la ou les adjonctions envisagées.

Le pH de la composition finale de traitement cosmétique est généralement compris entre 3 et 12 environ, et de préférence entre 5 et 11 environ. Il peut être ajusté à la valeur désirée au moyen d'agents acidifiants ou alcalinisants habituellement utilisés en cosmétique ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants, on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule suivante : 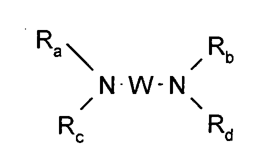 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition finale de traitement cosmétique peut se présenter sous des formes diverses, telles que sous forme de liquides, de crèmes, de gels ou sous toute autre forme appropriée pour réaliser un traitement des matières kératiniques, et notamment des cheveux humains.

La composition finale de traitement cosmétique peut être utilisée, par exemple, comme shampoing, après-shampoing, soin rincé
ou non rincé, masque de soin profond, gel douche, lotion ou crème de traitement des matières kératiniques.

La présente invention concerne aussi un procédé de traitement cosmétique des matières kératiniques, comprenant la préparation d'une composition de traitement cosmétique selon le procédé tel que défini ci-dessus, et son application sur les matières kératiniques, par exemple par l'intermédiaire d'un opérateur ou par l'intermédiaire d'un dispositif ne nécessitant pas d'intervention humaine. La durée d'application peut varier entre 15 secondes et 1 heure.

Avant application, la composition de traitement cosmétique obtenue selon le procédé de l'invention, peut être mélangée à un milieu cosmétiquement acceptable et/ou à un ou plusieurs additifs classiquement utilisés en cosmétique tels que décrits ci-dessus.

Un autre mode de réalisation consiste à préparer au moins deux compositions de traitement cosmétique selon le procédé de l'invention, à les mélanger, et à ajouter éventuellement un milieu cosmétiquement acceptable et/ou un ou plusieurs additifs classiquement utilisés en cosmétique tels que décrits ci-dessus, puis à appliquer la composition finale obtenue sur les matières kératiniques.

L'exemple ci-après illustre la présente invention.

### EXEMPLE

On mélange les ingrédients solides suivants dans les proportions indiquées en % en poids par rapport au poids total de mélange solide :
- Polymère cationique cellulosique associatif vendu sous la dénomination commerciale Quatrisoft LM 200 par la société AMERCHOL 8 %
- Mélange d'alcools linéaires (C18/C20/C22) vendu sous la dénomination commerciale Nafol 1822 par la société SASOL 4 %
- Lauroyl sarcosinate de sodium 80 % Polydiméthylsiloxane à groupement béhénate vendu sous la dénomination commerciale Mirasil Wax-B par la société RHODIA 8 %

On place 5 g. de ce mélange dans une machine expresso du commerce. On fait ensuite passer de la vapeur d'eau jusqu'à l'obtention d'une composition (A) présentant un volume final de 50 ml.

On obtient ainsi une composition de traitement cosmétique prête à être appliquée sur les cheveux.

On obtient des cheveux doux et un bon effet coiffant et fixant.

On peut ajouter deux parties en poids de composition (A), à une partie en poids d'une composition aqueuse (B) contenant 1 % en poids d'hydroxyéthylcellulose, pour faciliter l'application.

## Revendications

1. Procédé de préparation d'une composition de traitement cosmétique des matières kératiniques, **caractérisé en ce qu'**il comprend une étape de percolation de fluide comprenant au moins de la vapeur d'eau, sous une pression allant de 3 à 30 bars, au travers d'au moins un agent de conditionnement siliconé ou non, sous forme solide ou pâteuse, choisi parmi les alcools gras linéaires ou ramifiés ayant de 14 à 50 atomes de carbone, les hydrocarbures linéaires ou ramifiés, d'origine minérale ou synthétique, les cires, les composés de type céramide, et les gommes ou résines de silicone organomodifiées ou non.

2. Procédé selon la revendication 1, **caractérisé en ce que** le fluide est constitué par de la vapeur d'eau éventuellement accompagnée d'eau liquide, ou par un mélange de vapeur d'eau éventuellement accompagnée d'eau liquide, et d'un ou plusieurs solvants liquides et/ou gazeux cosmétiquement acceptables.

3. Procédé selon la revendication 1 ou 2, **caractérisé en ce que** les cires sont choisies parmi la cire d'abeille, le spermaceti, les cires fluorées et perfluorées, les cires de lanoline, de lanoline hydrogénée et de lanoline acétylée ; les cires de Candellila, d'Ouricury, de Carnauba, du Japon, le beurre de cacao, les cires de fibres de lièges ou de canne à sucre, la cire de son de riz, la cire de sapin, la cire de coton ; les cires microcristallines, la cire de paraffine, le pétrolatum, la vaseline, l'ozokérite, la cire de montan ; les huiles hydrogénées ayant une température de fusion supérieure à 40°C ; les cires de polyéthylène et les cires obtenues par synthèse de Fischer-Tropsch.

4. Procédé selon l'une quelconque des revendications précédentes, **caractérisé en ce que** les composés de type céramide sont choisis parmi :
- le 2-N-linoléoylamino-octadécane-1,3-diol,
- le 2-N-oléoylamino-octadécane-1,3-diol,
- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1,3-diol,
- le 2-N-stéaroyl amino-octadécane-1,3,4 triol,
- le 2-N-palmitoylamino-hexadécane-1,3-diol et
- le N-docosanoyl N-méthyl-D-glucamine.

5. Procédé selon l'une quelconque des revendications précédentes, **caractérisé en ce que** les résines et les gommes de silicone non organomodifiées sont choisies parmi les polydialkylsiloxanes, les polydiarylsiloxanes et les polyalkylarylsiloxanes.

6. Procédé selon la revendication 5, **caractérisé en ce que** les résines de silicone sont choisies parmi les composés siloxaniques réticulés renfermant les motifs :
R₂SiO_{2/2}, R₃SiO_{1/2}, RSiO_{3/2} et SiO_{4/2}
dans lesquels R représente un groupement hydrocarboné comportant de 1 à 16 atomes de carbone, ou un groupement phényle.

7. Procédé selon l'une quelconque des revendications précédentes, **caractérisé en ce que** les silicones organomodifiées sont des polyorganosiloxanes comportant des groupements polyéthylèneoxy et/ou polypropylèneoxy, des groupements thiols, des groupements hydroxylés, des groupements acyloxyalkyle, des groupements anioniques du type carboxylique, des groupements polyesters et/ou des groupements hydroxyacylamino.

8. Procédé selon l'une quelconque des revendications précédentes, **caractérisé en ce que** l'agent de conditionnement sous forme solide ou pâteuse est mis en oeuvre en mélange avec au moins un adjuvant.

9. Procédé selon la revendication 8, **caractérisé en ce que** l'adjuvant est choisi parmi les argiles, les sels, les tensioactifs anioniques, non ioniques, cationiques ou zwittérioniques, les épaississants naturels ou de synthèse, l'amidon éventuellement modifié, les billes de verre, la silice, le nylon, l'alumine, le dioxyde de titane, les zéolithes, le poly(méthacrylate de méthyle) (PMMA), le chitosane, la maltodextrine, la cyclodextrine, les mono- ou disaccharides, l'oxyde de zinc, de zirconium, les silicabades, le talc, l'acide polyaspartique, les borosilicates notamment de calcium, le polyéthylène, le coton, le polytétrafluoroéthylène (PTFE), la cellulose et ses dérivés, les composés superabsorbants, les carbonates de magnésium ou de calcium, les grains de maïs, le polyacrylamide, l'hydroxyapatite poreux, la soie, le collagène, la sciure de bois, la poudre de fucus, les extraits de blé, de riz, de pois, de lupin, de soja ou d'orge, la polyvinylpyrrolidone réticulée, l'alginate de calcium, le charbon actif, et les particules de poly(chlorure de vinylidène/acrylonitrile).

10. Procédé selon la revendication 8 ou 9, **caractérisé en ce que** l'agent de conditionnement est présent en une quantité allant de 0,5 à 99% en poids, de préférence de 1 à 80 % en poids par rapport au poids total agent(s) de conditionnement et adjuvant(s) sous forme solide ou pâteuse.

11. Procédé selon l'une quelconque des revendications 8 à 10, **caractérisé en ce que** la composition de traitement cosmétique obtenue contient outre le ou les agents de conditionnement et le(s) composant(s) du fluide, éventuellement tout ou partie du ou des adjuvants présents dans le mélange sous forme solide ou pâteuse.

12. Procédé selon l'une quelconque des revendications précéndentes, **caractérisé en ce que** l'étape de percolation est mise en oeuvre avec un fluide sous une pression d'au moins 10 bars.

13. Composition de traitement cosmétique des matières kératiniques susceptible d'être obtenue par le procédé selon l'une quelconque des revendications précédentes.

14. Composition de traitement cosmétique des matières kératiniques selon la revendication 13, ne comprenant pas de conservateur.

15. Procédé de traitement cosmétique des matières kératiniques, **caractérisé en ce que** l'on prépare une composition de traitement cosmétique par le procédé selon l'une quelconque des revendications 1 à 12, et que l'on applique cette composition sur les matières kératiniques.

16. Procédé de traitement cosmétique des matières kératiniques selon la revendication 15, **caractérisé en ce que** l'on applique cette composition sur les matières kératiniques par l'intermédiaire d'un dispositif ne nécessitant pas d'intervention humaine.

17. Procédé de traitement cosmétique des matières kératiniques selon la revendication 15, **caractérisé en ce que**, avant l'application, la composition de traitement cosmétique préparée selon le procédé selon l'une quelconque des revendications 1 à 12, est mélangée à un milieu cosmétiquement acceptable et/ou à un ou plusieurs additifs utilisés en cosmétique.

18. Procédé de traitement cosmétique des matières kératiniques, **caractérisé en ce que** l'on prépare au moins deux compositions de traitement cosmétique selon le procédé selon l'une quelconque des revendications 1 à 12, on les mélange et on applique le mélange sur les matières kératiniques.

19. Dispositif de conditionnement d'une composition cosmétique, comprenant un logement fermé délimité par au moins une paroi au moins en partie perméable au fluide sous pression d'au moins 3 bars, ladite composition contenant au moins un agent de conditionnement siliconé ou non, sous forme solide ou pâteuse.

20. Dispositif selon la revendication 19, **caractérisé en ce que** le logement est délimité par deux feuilles scellées.

21. Dispositif selon la revendication 19, **caractérisé en ce que** le logement est délimité par une barquette fermée par un couvercle.

22. Utilisation de la composition obtenue par le procédé selon l'une quelconque des revendications 1 à 12, pour le traitement cosmétique des matières kératiniques.

23. Utilisation selon la revendication 22, pour le conditionnement des cheveux.
